# EUROPEAN PATENT APPLICATION

(11) **EP 1 502 907 A1**
(43) Date of publication of application: **02.02.2005**
(21) Application number: 04103580.9
(22) Date of filing: 27.07.2004
(51) Int. Cl.: C07C 29/143, C07C 33/46

(54) **A process for the preparation of (2-hydroxymethyl-phenyl)-phenyl-methanol derivatives**

(30) Priority: 28.07.2003 IT FI20030202
(71) Applicant: Synteco S.p.A., 27028 S. Martino Siccomario (IT)
(72) Inventor: Angeli, Roberto, 50018 Scandicci (IT); Possenti, Marco, 50133 Firenze (IT)
(74) Representative: Gervasi, Gemma, Dr.

(57) **Abstract**

A process is described for the preparation of (2-hydroxymethyl-phenyl)-phenyl-methanol derivatives of general formula (I) 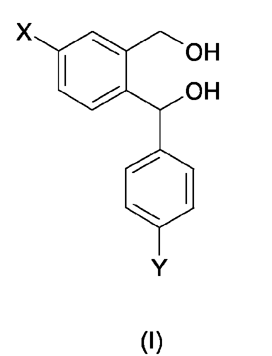 useful as intermediates in the synthesis of active pharmaceutical ingredients, comprising the reduction of the corresponding benzophenonic derivative with potassium borohydride.

## Description

### Field of the invention

The present invention relates to a process for the preparation of (2-hydroxymethyl-phenyl)-phenyl-methanol derivatives of general formula (I) reported below, useful as intermediates in the synthesis of active pharmaceutical ingredients.

### State of the art

To date, various processes for the preparation of 1-dimethylaminopropyl-1-phenyl-phthalans of formula (A) have been reported in the literature

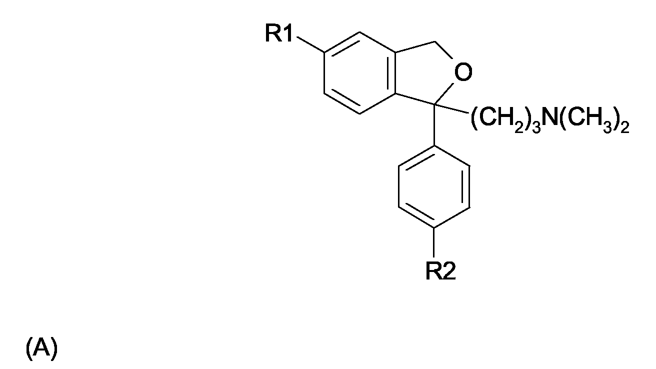

The synthesis of those compounds is indeed of great interest, since they are products which have been used for long time as active ingredients with anti-depressive action. Amongst those, one product which is of particular interest due to its high activity and scarcity of side effects, is 1-[3-(dimethylamino)propyl]-1-(4-fluorophenyl)-5-phthalancarbonitrile, i.e. the compound of formula (A) wherein R₁ is a CN group and R₂ is fluorine, known as Citalopram.

Many of the processes for preparing those compounds foresee a synthetic scheme which passes through intermediates having the following formula (B) 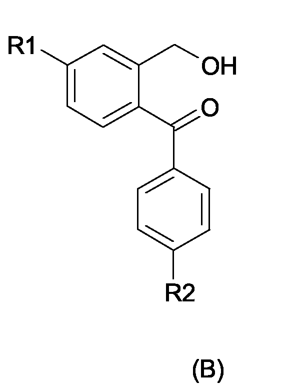 wherein R₁ and R₂ are halogen atoms, obtained through the reduction of the corresponding benzophenonic derivatives of formula (C) 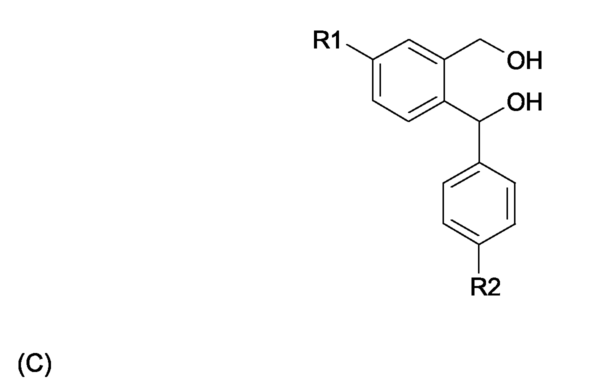 wherein R₁ and R₂ are halogen atoms.

In US Patent No. 4,136,193, for example, which describes the compounds of formula (A) for the first time; a preparation process for those compounds, comprising the reduction of compounds of formula (C) to the above reported compounds of formula (B) is illustrated. Still according to US Patent No. 4,136,193 such reduction is carried out by making an ethereal solution of benzophenone (C) react with lithium aluminium hydride in ether under reflux.

The use of lithium aluminium hydride, which is an extremely powerful reducing agent, has a series of drawbacks, amongst which the high cost, which makes it useful only for small scale laboratory applications, and the dangers implicit in its use: the dry, crystalline lithium aluminium hydride powder produces a dust which is highly irritating for the mucosa; furthermore, it can spontaneously ignite and violently explode if heated beyond 120°C. The scalability of this reaction, under the operating conditions described in the known art, is hence compromised, both due to the overly high costs of the reagent and due to the necessary precautions regarding its use.

There is still therefore the need for a process of preparation of di-hydroxy derivatives of formula (B), which allows overcoming the drawbacks illustrated above for the processes of the known art, and can be easily scaled up.

### Summary of the invention

Now the Applicant has found a process for the preparation with high yields of (2-hydroxymethyl-phenyl)-phenyl-methanol derivatives of formula (I) reported hereinafter, which may be easily reproduced on a large scale and allows overcoming the drawbacks of the known art, thanks to the use of potassium borohydride as reducing agent.

It is therefore subject of the present invention a process for the preparation of (2-hydroxymethyl-phenyl)-phenyl-methanol derivatives of general formula (I) 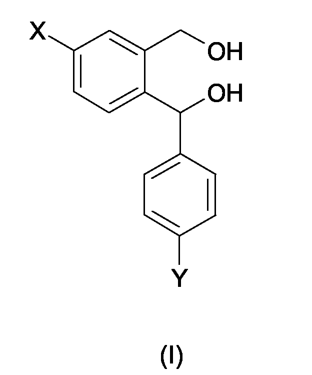 wherein X and Y are halogens,
comprising the reduction of the corresponding benzophenonic derivative of general formula (II) with potassium borohydride 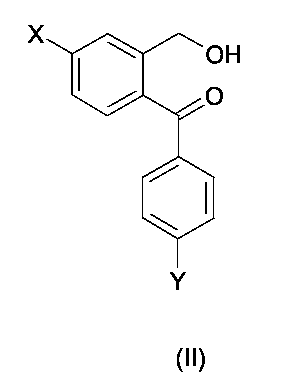 wherein X and Y are as defined above.

### Detailed description of the invention

Reduction of the benzophenone of formula (II) according to the present process may be carried out in one or more suitably selected organic solvents, at a temperature ranging between -10°C and 30°C, preferably at a temperature higher than 0°C, and more preferably at room temperature.

Preferably the amount of potassium borohydride added is such that the molar ratio between the compound of formula (II) and the borohydride ranges between 1:1 and 1:1.5, and more preferably is equal to 1:1.15.

In the process according to the present invention, the reduction reaction is preferably carried out in an organic solvent selected from the lower alcohols (having from 1 to 5 carbon atoms), and preferably in an alcoholic solvent selected from the group consisting of ethanol, methanol, and isopropyl alcohol; optionally, the reaction is carried out in the presence of a further non-alcoholic organic solvent, for example tetrahydrofuran.

According to a particularly preferred embodiment of the present invention the alcoholic solvent used in the present process is ethanol.

The amount of alcoholic solvent used in the present process is such that the weight/volume ratio between the alcoholic solvent and the potassium borohydride is preferably 1/10.

The starting compounds of formula (II) may be prepared according to procedures known to any person skilled in the art, for example by the reaction of a suitably substituted phenylmagnesium halide, with a suitably substituted isobenzofuran-1-one, as described for example in the US Patent No. 4,136,193.

According to a preferred embodiment of the invention, the present process relates to compounds of formula (I) and (II) wherein X is Br and Y is F.

The present process allows the attainment of the compound of formula (I) with such a degree of purity as to be able to be used directly for the synthesis of products of interest, such as for example the above mentioned compounds of formula (A). The compound of formula (I) wherein X is Br and Y is F, for example, may be used as an intermediate in the synthesis of citalopram, by following for example the synthetic procedure described in US Patent No. 4,136,193, or other methods known to any person skilled in the art.

The use of potassium borohydride under the above described conditions makes the present process particularly advantageous with respect to known processes in the art, since this is a reagent which has much lower costs than lithium aluminium hydride; and does not require the same precautions for use that are necessary when reduction is carried out using lithium aluminium hydride. Furthermore, despite not having the reducing power of lithium aluminium hydride, potassium borohydride allows the attainment of the reduction product with high yield, even performing the reaction at room temperature.

The following examples are reported for the purposes of non limiting illustration of the present invention.

### EXAMPLE 1

### Preparation of the compound of formula (I) wherein X is Br and Y is F

150 g (0.486 mol) of 4-bromo-4-fluoro-2-hydroxymethyl-benzophenone have been dissolved in 400 ml of tetrahydrofuran. To the solution thus obtained has been added a suspension of 30 g of potassium borohydride in 300 ml of ethanol, maintaining the temperature below 25°C.

The reaction mixture has been kept under stirring for 2 hours at room temperature and has then been quenched by the addition of 1000 ml of an aqueous solution of dilute hydrochloric acid.

The solvents have been removed from the reaction mixture by evaporation under vacuum and the resulting aqueous solution has been extracted three times with toluene.

The toluene phases have been desiccated over anhydrous sodium sulphate and evaporated under vacuum thus obtaining a crude oily residue (120 g; 0.386 mol) which was 4-bromo-(2-hydroxymethyl)-phenyl-(4-fluorophenyl)-methanol (yield = 79.4%).

## Claims

1. A process for the preparation of (2-hydroxymethyl-phenyl)-phenyl-methanol derivatives of general formula (I) 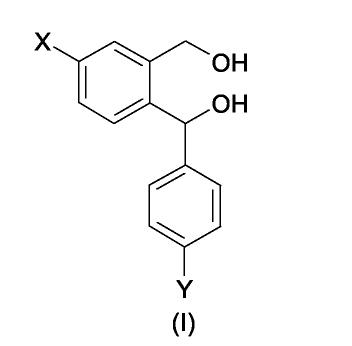 wherein X and Y are halogens,
comprising the reduction of the corresponding benzophenonic derivative of general formula (II) with potassium borohydride 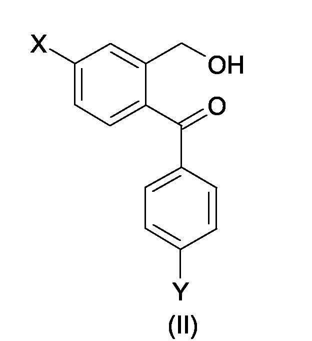 wherein X and Y are as defined above.

2. The process according to claim 1, wherein X is Br and Y is F.

3. The process according to claim 1, wherein the molar ratio between the compound of formula (II) and potassium borohydride ranges between 1:1 and 1:1.5.

4. The process according to claim 3, wherein the molar ratio between the compound of formula (II) and potassium borohydride is equal to 1:1.15.

5. The process according to claim 1, wherein said reduction reaction is carried out in an alcoholic solvent selected from the group consisting of lower alcohols, optionally in the presence of a further non-alcoholic organic solvent.

6. The process according to claim 5, wherein said lower alcohols are selected from the group consisting of methanol, ethanol, and isopropyl alcohol.

7. The process according to claim 6, wherein said lower alcohol is ethanol.

8. The process according to claim 5, wherein the weight/volume ratio between the potassium borohydride and said alcoholic solvent is 1/10.

9. The process according to claim 5, wherein said non-alcoholic organic solvent is tetrahydrofuran.

10. The process according to claim 1, wherein said reduction reaction is carried out at a temperature ranging between -10°C and 30°C.

11. The process according to claim 10, wherein said reduction reaction is carried out at a temperature higher than 0°C.

12. The process according to claim 10, wherein said reduction reaction is carried out at room temperature.
